Europäisches Patentamt

European Patent Office

Office européen des brevets

(11) Publication number: **0 311 297**
**A2**

## (12) EUROPEAN PATENT APPLICATION

(21) Application number: 88309015.1

(22) Date of filing: 29.09.88

(51) Int. Cl.⁴: **C07C 29/32 , C07C 31/08 ,**
**C07C 31/10 , C07C 31/12**

(30) Priority: 08.10.87 GB 8723654

(43) Date of publication of application:
**12.04.89 Bulletin 89/15**

(84) Designated Contracting States:
**DE FR NL**

(71) Applicant: **Coal Industry (Patents) Limited**
**Hobart House Grosvenor Place**
**London SW1X 7AE(GB)**

(72) Inventor: **Gavin, Derek Gabriel**
**11 The Napping**
**Longhope Gloucestershire(GB)**

(74) Representative: **Wood, John Irwin**
**NATIONAL COAL BOARD Hobart House**
**Grosvenor Place**
**London SW1X 7AE(GB)**

(54) **Alcohol synthesis process.**

(57) Ethanol, propanol and butanol may be made in good yields by reacting a lower alcohol such as methanol with a gas containing CO and $H_2$ in the ratio 20:80 to 0:100 by volume $H_2$ to CO, recycling lower alcohol and maintaining a CO partial pressure of at least 30 bar at the reactor exit.

COMPOSITION DIAGRAM

FIG. 1

EP 0 311 297 A2

## ALCOHOL SYNTHESIS PROCESS

This invention concerns an alcohol synthesis process, more especially a process for synthesising higher alcohols substantially free of methanol from carbon monoxide, methanol or other lower alcohols and hydrogen.

It is now recognised that the use of lead alkyls as octane enhancers in gasoline are undesirable because of contamination of the environment by toxic lead compounds. Favoured alternatives to lead alkyls are oxygenated organic compounds such as mixed alcohols and methyl tertiary butyl ether.

Alcohols which are useful as antiknock agents are ethanol, propanol and butanol and mixtures thereof. Methanol improves octane ratings of gasoline but has well-known drawbacks including its toxicity, its ability to increase the vapour pressure of the gasoline and to reduce the distillation boiling points of lighter gasoline components and its adverse effect on the tolerance of gasoline to water before phase separation occurs. The problems exhibited by methanol are of sufficient seriousness that legislation is being considered to limit its use to below 3% by wt of gasoline in Europe and in USA. It would be advantageous to make mixed alcohols containing no or substantially no methanol suitable for blending into gasoline or as chemicals in their own right, from carbon monoxide, hydrogen if necessary, and methanol in one stage with yields which are commercially economical under conditions which are commercially viable in terms of pressure and temperature.

It is known to make mixed alcohols from synthesis gas, by passing the gas over a catalyst. A variety of catalysts have been disclosed, mainly based on iron oxide, copper with cobalt, rhodium or molybdenum. For example, the Synol process employed an iron oxide/ potassium oxide catalyst to produce a mixture of alcohols (55%) and hydrocarbons (40%). The mixture boiled in the range $34^\circ$ to $360^\circ$C, making them unsuitable for blending into modern gasoline. More recent processes published in the patent literature have concentrated on promoted rhodium catalysts, mainly on silica supports. Rhodium is, however, extremely expensive and is not particularly selective for producing alcohols at high rates because low value hydrocarbons are simultaneously produced.

It is also known to use molybdenum sulphide as a catalyst for making liquid hydrocarbons and organic oxygenates. For example, USP 2,490.488 discloses $MoS_2$ modified by the addition of alkali metal compounds. European Patent Application No.0119609 (Dow) discloses a number of catalysts, both supported and bulk catalysts, for example 9 and 18 relate to $MoS_2$ promoted with potassium carbonate. Example 9 discloses 32% $MoS_2$ and 7.5% $K_3CO_3$ on active carbon and dried at $300^\circ$C in nitrogen yielding 121g of alcohols/h/kg catalyst at a selectivity of 91.6% ($CO_2$ -free basis). Example 18 discloses a clay-supported catalyst containing 66% $MoS_2$ and 10% $K_2CO_3$ which yields 367g of alcohols ·h/kg catalyst at 87% selectivity. The alcohols contained a substantial quantity of methanol.

Another recent European Application, No. 0 149 255 (Union Carbide), also discloses the production of alcohols using alkali-promoted $MoS_2$. The best results were disclosed in Examples 8 and 21, both giving a yield of 256g of alcohol containing methanol per hour per kg of catalyst at 80% selectivity using unsupported $MoS_2$ with cesium promoters. A mixture of unsupported $MoS_2$ and KOH gave 221g/h/kg at 80% selectivity.

USP 4,243,553 discloses the decomposition of ammonium thiomolybdate to yield high surface area $MoS_2$ for use in water gas shift and methanation reactions. No mention is made of alcohols in this document, but it is noted that reaction temperatures are relatively high.

Our co-pending UK Patent Application 2 185907 describes an improved catalyst based on $MoS_2$ and $K_2CO_3$ on an active carbon carrier and which is capable under optimum conditions of giving high yields of mixed alcohols containing methanol, eg up to 700 g/h/kg catalyst with selectivities of from 85 to 95% alcohols by weight on a $CO_2$ - free basis. The teaching of said Application is incorporated herein by reference.

Our co-pending UK Patent Application No.8617170 describes a process for making mixed alcohols substantially free of methanol, comprising passing synthesis gas defined as a mixture of hydrogen and carbon monoxide over an alcohol synthesis catalyst, in the presence of methanol, and fractionating the liquid product of the alcohol synthesis into a fraction containing ethanol, propanol and butanol and a fraction containing methanol and recycling said fraction containing methanol through the synthesis reactor, whereby there is substantially no net production of methanol. The invention is feasible because the methanol in the feedstock to the synthesis reactor prevents methanol being formed from hydrogen and carbon monoxide because the extent of this reaction is controlled by chemical equilibrium. The teaching of said application is incorporated herein by reference.

2

The disadvantages of making higher alcohols free of methanol by recycling the methanol using $H_2$ to CO ratios as low as 2:1 or 1:1 synthesis gas are the relatively low yields of higher alcohols and also the methanol in the reactor leads to an increase in methane formation thereby reducing the selectivity to higher alcohols. For example, using the aforementioned $MoS_2$ catalyst at 2:1 $H_2$:CO gas, yields of alcohols including methanol were 680g/h/kg catalyst. Whereas with methanol recycle and no net production of methanol, higher alcohol yields were only 100g/h/kg catalyst.

The present invention provides a process for the production of ethanol, propanol, butanol and higher alcohols with substantially no net production of methanol, comprising reacting a lower alcohol feed and a gas containing carbon monoxide and hydrogen with a composition in the range 20:80 to 0:100 by volume of hydrogen to carbon monoxide, over a heterogeneous solid phase alcohol synthesis catalyst under alcohol synthesis conditions, recycling unreacted alcohol feed and carbon monoxide and hydrogen after separation from the product higher alcohols, and maintaining a carbon monoxide partial pressure of at least 30 bar at the reactor exit.

The present invention can be described as an heterogeneously catalysed homologation reaction in which an alcohol such as methanol is reacted with carbon monoxide to increase its molecular weight to give for example ethanol, propanol and butanol. There are other processes described for homologating methanol, for example in European Patents 84 8331B and 13464A, but these are homogeneously catalysed in the liquid phase and require high pressure using catalysts based on ruthenium and cobalt.

It will be understood that in the context of the present invention, "higher" alcohols relate to an alcohol having a carbon number one more higher than that of the feedstock alcohol. Thus ethanol and propanol may be higher alcohol products when methanol and ethanol, respectively, are used as feedstock lower alcohols. Hereinafter, methanol will be generally referred to as the lower alcohol feedstock, but the invention is not to be considered as being limited thereto.

The nature of the process invented can be better understood in terms of the reaction equations. The stoichiometry of the reactions occurring in the production of higher alcohols can be represented by at least three sets of equations and the water gas shift reaction whereby water is converted to $CO_2$ and $H_2$ by reaction with CO. $CO + 2H_2 = CH_3OH$    (1)

(i)    $3CO + 3H = C_2H_5OH + CO_2$    (2)
$5CO + 4H_2 = C_3H_7OH + 2CO_2$    (3)
$7CO + 5H_2 = C_4H_9OH + 3CO_2$    (4)
$2CO + 2H_2 = CH_4 + CO_2$    (5)

(ii)    $CH_3OH + 2CO + H_2 = C_2H_5OH + CO_2$    (6)
$C_2H_5OH + 2CO + H_2 = C_3H_7OH + CO_2$    (7)
$C_3H_7OH + 2CO + H_2 = C_4H_9OH + CO_2$    (8)
$CH_3OH + CO = CH_4 + CO_2$    (9)

(iii)    $2CH_3OH + CO = C_2H_5OH + CO_2 + H_2$    (10)
$C_2H_5OH + CH_3OH + CO = C_3H_7OH + CO_2 + H_2$    (11)
$C_3H_7OH + CH_3OH + CO = C_4H_9OH + CO_2 + H_2$    (12)
$CH_3OH + CO = CH_4 + CO_2$    (13)

Tests have provided evidence that equations (10) to (13) describe the chemical reactions leading to higher alcohols rather than the reaction of carbon monoxide with hydrogen (2) to (5) or the reaction of carbon monoxide and hydrogen with alcohols (6) to (9). For example, tracer tests using $C^{13}H_3OH$ showed that most of the $C^{13}$ found its way into the product $C_2H_5OH$ indicating that $C_2H_5OH$ was formed from $CH_3OH$. The $CO_2$ produced came from the CO in the gas phase. When the concentration of carbon monoxide in the reaction was increased, the rate of production of propanol and butanol increased. When the concentration of methanol was increased in particular with a high concentration of carbon monoxide, then the rate of production of propanol and butanol increased. When methanol and hydrogen were reacted together, in the absence of carbon monoxide, no higher alcohols were produced. When ethanol was reacted with carbon monoxide, the proportion of propanol increased.

It is a feature of the invention that improved yields of higher alcohols are attained by maximising the concentration of methanol and carbon monoxide in the reaction zone of the synthesis reactor. This is achieved by operating at CO to $H_2$ ratios in the range 80:20 to 100:0. The hydrogen content can be such as to prevent the methanol decomposing by the reaction (1) above. It is found that the higher the methanol content of the feed, the less additional methanol is formed by the same reaction.

Certain advantages follow, according to the invention, by operating with a feed comprising CO-rich gas

EP 0 311 297 A2

and methanol, compared, for example, with the usually recommended 1:1 or 2:1 $H_2$:CO synthesis gas and methanol. The rate of production of higher alcohols depends on CO partial pressure. It is easier to achieve high CO partial pressures using CO-rich gas (80% CO and above on a methanol-free basis) than with hydrogen rich gas (33-50%CO). It is also found that product selectivity to higher alcohols improves because the rate of the methane forming reaction (see equation 5 above), is reduced by reducing the hydrogen concentration.

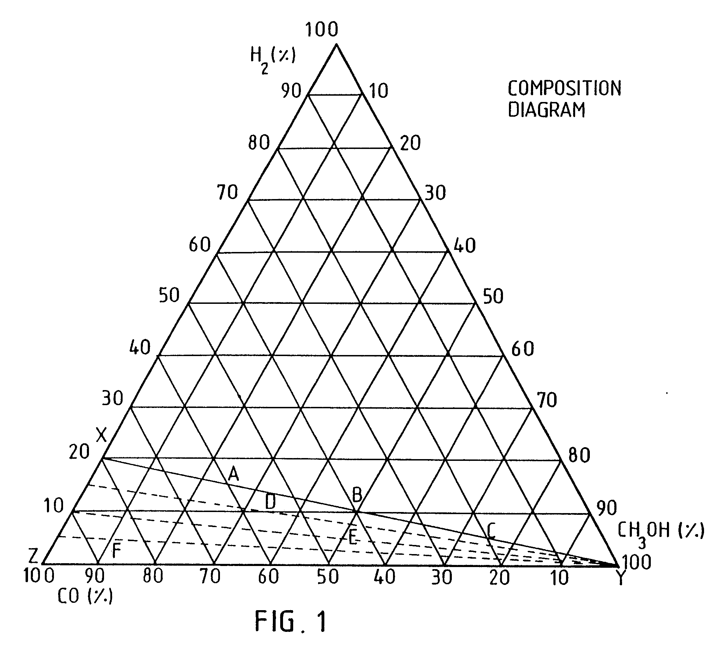

The activities of methanol, carbon monoxide and hydrogen depend on their proportions expressed as percentages and on the total pressure. The limits on the proportions of the methanol, carbon monoxide and hydrogen may be defined by reference to the accompanying Figure 1 which is a composition diagram for a feed stock for the process of the invention. The limits in terms of volume concentrations are 20% $H_2$, 100% $CH_3$ $OH$ and 100% CO, ie the area bounded by XY and Z. By way of example only, points A to F in Figure 1 may be considered. The volume proportions of the three reactants would be as follows:

|   | $CH_3OH$ (%) | CO (%) | $H_2$ (%) | $H_2$:CO (molar ratio) |
|---|---|---|---|---|
| A | 25 | 60 | 15 | 20:80 |
| B | 50 | 40 | 10 | 20:80 |
| C | 75 | 20 | 5 | 20:80 |
| D | 35 | 55 | 10 | 15:85 |
| E | 50 | 45 | 5 | 10:90 |
| F | 10 | 86 | 4 | 5:95 |

It can be seen that the line ABC represents a constant $H_2$:CO molar ratio, and the broken lines also represent constant $H_2$:CO molar ratios.

In terms of pressure, experiments indicate that the minimum partial pressure of CO at the reactor exit to obtain reasonable rates of reaction is 30 bar. The upper limit of total pressure depends on commercially available reactors which are economical to construct. A preferred range of CO partial pressure is 40 to 300 bar. The hydrogen pressure preferred range is defined with respect to the $H_2$:CO molar ratios in the range of 20:80 to 0:100. The preferred concentration of methanol depends on the activity of the catalyst and the reaction temperature. The range of lower alcohol fed into the reactor can be expressed as a liquid hourly space velocity. A preferred range for methanol is 0.1 to 10 litres/hour/litre of catalyst; a desirable range is is 0.2 to 5 litres/hour/litre of catalyst.

Although the methanol concentration is defined within the boundary limits XYZ in Fig.1, the methanol equilibrium depends upon temperature and pressure, and the equilibrium concentration of the methanol can be calculated from thermochemical data.

Diluent gases may be present in the feed gases, eg. carbon dioxide, methanol, water vapour and nitrogen. The concentration of such diluent gases should be taken into account when defining the partial pressures of main reactants. $H_2S$ can be present in the feed gas when using the preferred catalyst based on $MoS_2$, but should be absent when using a metallic catalyst such as those based on Cu, Co or Rh.

The gas flow or gas recycle rate should be considered in optimising process conditions. For example, if the gas flow is low, the products of reaction dilute the reactants, reducing the partial pressures and hence reducing the rate of reaction. The process can be operated at preferred gas flows expressed as gaseous hourly space velocities of 500 to 50,000 $h^{-1}$ and especially 2,000 to 20,000 $h^{-1}$.

Reaction temperature is significant for a variety of reasons. A preferred temperature range is 240° to 350°C. At the higher end of the temperature range, methanol production increases leading to poor selectivities and loss of higher alcohols. Also at higher temperatures, methanol tends to decompose to hydrogen and carbon monoxide and is less effective in reacting with carbon monoxide to give higher alcohols. At the low end of the temperature range, molybdenum carbonyl $Mo(CO)_6$, which is a white crystalline solid, can form when using $MoS_2$ catalyst, particularly under high partial pressures of CO, leading to Mo loss from the catalyst. Relatively high partial pressures of methanol in the feedstock and temperature of 260°C and above reduce the formation of molybdenum carbonyl. For these reasons, an especially preferred temperature range is 260 to 310°C.

Suitable catalysts for use in the present invention include those known in the art as alcohol synthesis catalysts, such as iron oxide, copper with cobalt, copper promoted with potassium carbonate and rhodium, or molybdenum sulphide promoted with an alkali metal, eg promoted with potassium carbonate. Our UK Application 2 185 907 European Patent Application No 0235886) describes highly active catalysts based on molybdenum sulphide absorbed on to particulate active carbon and promoted by potassium carbonate.

4

Catalysts based on this formulation are preferred for use in the present invention for the following reasons:

a) Mixed alcohol yields including methanol of 680g/h/kg catalyst have been found under suitable conditions;

b) High selectivities to alcohols including methanol compared to $CH_4$ + $C_2H_6$ were found, for example in the range 83 to 95 wt% ($CO_2$ free basis);

c) the catalyst has a high water gas shift capacity so that most of the excess combined oxygen is eliminated as $CO_2$ instead of as $H_2O$, leading to an alcohol product needing little dewatering.

d) The catalyst is highly active for equilibrating with $H_2$ and CO.

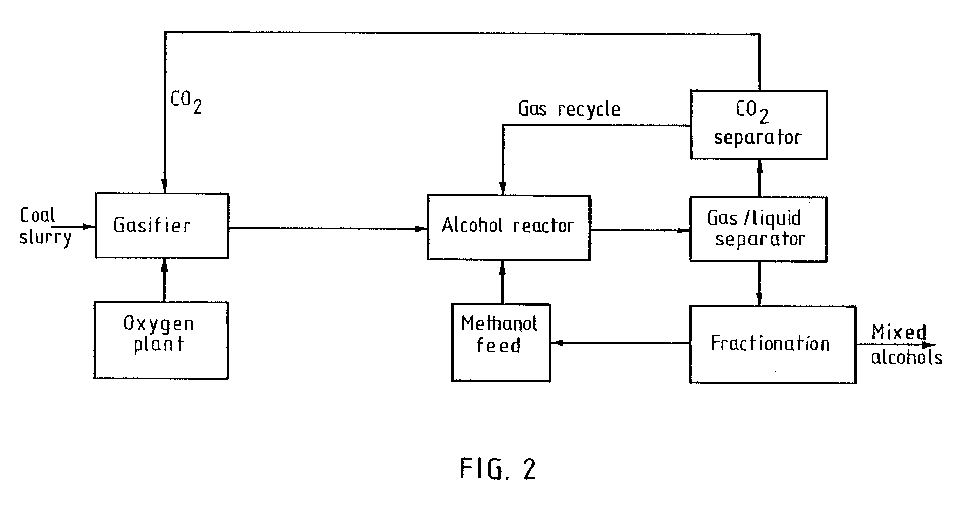

The invention may be more fully described with reference to the accompanying Fig.2, which is a schematic Flow Diagram of one embodiment of a process according to the present invention.

A carbon monoxide-rich gas is produced in the gasifier from oxygen, steam or carbon dioxide and any carbonaceous feedstock such as coal, heavy oil or by the partial oxidation of natural gas. Other means of producing the carbon monoxide-rich gas include the steam reforming of natural gas, LPG or naphtha or by methanol decomposition. A means of separating hydrogen from the gas, thereby enriching it with carbon monoxide, may be necessary depending on the method of manufacture of the synthesis gas. Separated hydrogen could be used in the manufacture of the methanol which is the second major reactant.

Carbon monoxide-rich gas containing between 20 and 0% hydrogen and methanol is fed to the alcohol synthesis reactor containing the synthesis catalyst.

The synthesis reactor can be one of a variety of designs, eg. a tubular or squat fixed bed, moving bed or fluidised bed, or an inert liquid recycling reactor. Because of the nature of the process, some care needs to be taken in the reactor design to ensure adequate heat removal and temperature control, but this lies within the expertise of the competent chemical engineer. Make up and recycled synthesis gas are co-fed with methanol into the reactor. The products from the synthesis reactor are condensed and passed to a gas-liquid separator; the resulting gas stream is further separated into $CO_2$ which is optionally recycled to the gasifier or may be discarded, hydrocarbon gases in small amount which are removed in a bleed gas (not shown) and unreacted synthesis gas which is recycled to the synthesis reactor. The liquid stream from the gas-liquid separator containing the condensed alcohols is passed to a fractionating column from which all the methanol produced is recycled to the synthesis reactor and a mixed alcohol product containing ethanol, propanol and butanol is produced, which may contain small quantities of water. The proportions of the alcohols depend upon the $H_2$:CO ratio and the reaction conditions. After separation of methanol, there may be 10 to 30% ethanol, 20 to 30% propanol and 50 to 70% butanol. The invention is not limited by the compositions but there is an advantage in the process in that the composition of the mixed alcohols can be varied by adjusting the hydrogen content of the feed gas and the methanol feedrate.

The invention will now be described by way of example only. The principle of the process was proved in tests carried out in an experimental unit.

Carbon monoxide-rich gas was prepared by blending mixtures of pure carbon monoxide and hydrogen; it was fed to a 15 ml reactor immersed in an electrically heated fluidised sand bath. Methanol was pumped by a high pressure liquid pump to the reactor. The reactor was heated to 300°C and in tests, a pressure of 100 bar and a GHSV of 3000 $h^{-1}$ were used. The liquid products were cooled, condensed and collected in a high pressure separator, weighed and analysed. The gas was depressurised, its volume was measured and its composition determined. From the weights, volumes and analysis of feed and products, mass balances were determined. From the mass balances, the feed rates in terms of kg/h/kg of catalyst of $H_2$, CO and $CH_3OH$ and the product rates of $H_2$, CO, $CH_3OH$, $C_2H_5OH$, $C_3H_7OH$, $C_4H_{10}OH$, $CH_4$ and $CO_2$ were determined.

The liquid products generally contained approximately 2-3% by weight of water. From the feed and product rates, the CO and $CH_3OH$ conversions (%) and yields (kg/h/kg catalyst) were determined. Selectivity expressed as g-atoms of carbon in the higher alcohols divided by the combined g-atoms of carbon in the higher alcohols and the g-atoms carbon in the hydrocarbon gas product was estimated as a percentage, and on a $CO_2$ - free basis.

The catalyst used in the following four examples had a loading of 24% $MoS_2$ and 7% $K_2CO_3$ on a coconut shell active carbon carrier and was prepared as described in Example 2 of our U.K. patent Application 2 185 907.

Example 1 (Comparison only)

The reactor was operated on synthesis gas at a 50:50 $H_2$:CO ratio with increasing quantities of $CH_3OH$

pumped into the reactor. Yields of higher alcohols were in the range 0.11 to 0.19 kg/h/kg of catalyst. With zero $CH_3OH$ addition (sample 1), 0.24 kg of $CH_3OH$ was made and finally some of the added $CH_3OH$ decomposed. Also $H_2$ was used up in the reactions. Selectivity decreased to 40% in terms of higher alcohols as more $CH_3OH$ was added.

Example 2

The reactor was operated using a gas at a 20:80 $H_2$:CO ratio, again with increasing amounts of $CH_3OH$. It can be seen that yields of higher alcohols increased to 0.47 kg/h/kg catalyst. $CH_3OH$ was consumed in the process and a small quantity of $H_2$ was liberated. Selectivity to higher alcohols relative to $CH_4$ improved to 74% with increased $CH_3OH$ additions. Hence at higher CO concentrations, improved yields and selectivities may be achieved in addition to a $CH_3OH$- free product. A small amount of methyl acetate was found in the alcohol product.

Example 3

The reactor was operated at 0:100% $H_2$:CO at the inlet, again with increasing amounts of $CH_3OH$. Higher alcohol yields of 0.43 kg/h/kg catalyst were achieved at a selectivity of 63% but approximately twice the amount of $CH_3OH$ was consumed compared with Example 2, it is thought because some of the $CH_3OH$ decomposed to $H_2$ and CO instead of reacting to higher alcohols.

Example 4

The reactor was operated at 125 bar at a $H_2$:CO ratio of 10:90, with increasing $CH_3OH$ additions. Higher alcohol yields of 0.6 kg/h/kg catalyst were obtained at 82% selectivity. Most of the $CH_3OH$ reaction would be accounted for by reaction to higher alcohols, $CH_4$ and $CO_2$ with minimum decomposition to $H_2$ and CO.

## COMPARATIVE EXAMPLE 1

| Sample No. | | 1 | 2 | 3 | 4 | 5 |
|---|---|---|---|---|---|---|
| Methanol L.H.S.V. $(h^{-1})$ | | 0 | 0.4 | 0.6 | 0.80 | 1.2 |
| G.H.S.V. $(h^{-1})$ | | 3100 | 3100 | 3100 | 3000 | 3500 |
| **Reactor inlet** | | | | | | |
| $H_2$:CO | | 50:50 | 50:50 | 50:50 | 50:50 | 50:50 |
| $H_2$ | (Vol %) | 50 0 | 47.1 | 45.9 | 44.4 | 42.9 |
| CO | (Vol %) | 50.0 | 47.1 | 45.9 | 44.4 | 42.9 |
| $CH_3OH$ | (Vol %) | 0 | 5.6 | 8.2 | 11.2 | 13.9 |
| **Feed rate (kg/h/kg cat.)** | | | | | | |
| $H_2$ | | 0.28 | 0.28 | 0.28 | 0.26 | 0.31 |

| | | | | | |
|---|---|---|---|---|---|
| CO | 3.92 | 3.92 | 3.92 | 3.70 | 4.34 |
| $CH_3OH$ | 0.0 | 0.53 | 0.80 | 1.07 | 1.60 |

**Product rate** (kg/h/kg cat.)

| | | | | | |
|---|---|---|---|---|---|
| $H_2$ | 0.22 | 0.23 | 0.24 | 0.23 | 0.25 |
| CO | 3.25 | 3.22 | 3.25 | 3.08 | 3.67 |
| $CH_3OH$ | 0.24 | 0.58 | 0.84 | 0.99 | 1.55 |
| $C_2H_5OH$ | 0.07 | 0.10 | 0.11 | 0.11 | 0.09 |
| $C_3H_7OH$ | 0.02 | 0.03 | 0.04 | 0.04 | 0.04 |
| $C_4H_9OH$ | 0.02 | (0.07) | 0.03 | 0.04 | 0.02 |
| $CH_4$ | 0.05 | 0.09 | 0.11 | 0.12 | 0.16 |
| $CO_2$ | 0.24 | 0.36 | 0.41 | 0.41 | 0.52 |

**Conversions %**

| | | | | | |
|---|---|---|---|---|---|
| CO | 17 | 18 | 17 | 17 | 16 |
| $CH_3OH$ | -ve | -8 | -4 | 7 | 3 |

**Yields** (kg/h/kg cat.)

| | | | | | |
|---|---|---|---|---|---|
| Higher alcohols | 0.11 | 0.20 | 0.18 | 0.19 | 0.15 |
| $CH_3OH$ | 0.24 | 0.05 | 0.04 | -0.08 | -0.05 |
| $H_2$ | -0.06 | -0.05 | -0.04 | -0.03 | -0.06 |
| $CO_2$ | 0.24 | 0.36 | 0.41 | 0.41 | 0.52 |

| | | | | | |
|---|---|---|---|---|---|
| **Selectivity** (molar %) | 82* | 65* | 63* | 53 | 40 |

**Alcohol compositions** (wt%)

| | | | | | |
|---|---|---|---|---|---|
| $CH_3OH$ | 68 | 20 | 18 | 0 | 0 |
| $C_2H_5OH$ | 20 | 40 | 50 | 58 | 60 |
| $C_3H_7OH$ | 6 | 12 | 18 | 21 | 27 |
| $C_4H_9OH$ | 6 | 28 | 14 | 21 | 13 |

\* to total alcohols.

EXAMPLE 2

| Sample No. | | 1 | 2 | 3 | 4 | 5 |
|---|---|---|---|---|---|---|
| Methanol L.H.S.V.($h^{-1}$) | | 0.35 | 0.70 | 1.06 | 1.42 | 1.76 |
| G.H.S.V.($h^{-1}$) | | 3100 | 3300 | 3400 | 3300 | 3400 |
| **Reactor inlet** | | | | | | |
| $H_2$:CO | | 20:80 | 20:80 | 20:80 | 20:80 | 20:80 |
| $H_2$ | (Vol %) | 18.9 | 18.3 | 17.2 | 16.8 | 16.2 |
| CO | (Vol %) | 75.9 | 72.6 | 70.0 | 66.5 | 64.4 |
| $CH_3OH$ | (Vol %) | 5.2 | 9.1 | 12.8 | 16.7 | 19.4 |
| **Feed rate** (kg/h/kg cat.) | | | | | | |
| $H_2$ | | 0.11 | 0.12 | 0.12 | 0.12 | 0.12 |
| CO | | 6.19 | 6.55 | 6.72 | 6.55 | 6.83 |
| $CH_3OH$ | | 0.47 | 0.94 | 1.41 | 1.89 | 2.35 |
| **Product rate** (kg/h/kg cat.) | | | | | | |
| $H_2$ | | 0.12 | 0.14 | 0.15 | 0.16 | 0.16 |
| CO | | 5.80 | 6.10 | 6.19 | 6.16 | 6.61 |
| $CH_3OH$ | | 0.31 | 0.60 | 0.96 | 1.24 | 1.43 |
| $C_2H_5OH$ | | 0.05 | 0.06 | 0.06 | 0.06 | 0.06 |
| $C_3H_7OH$ | | 0.04 | 0.05 | 0.08 | 0.08 | 0.09 |
| $C_4H_9OH$ | | 0.08 | 0.13 | 0.19 | 0.26 | 0.32 |
| $CH_4$ | | 0.06 | 0.11 | 0.13 | 0.13 | 0.13 |
| $CO_2$ | | 0.29 | 0.47 | 0.53 | 0.51 | 0.56 |

EXAMPLE 2    contd.

Conversions (%)

| | | | | | |
|---|---|---|---|---|---|
| CO | 6 | 7 | 8 | 6 | 3 |
| $CH_3OH$ | 34 | 36 | 32 | 34 | 39 |

Yields (kg/h/kg cat.)

| | | | | | |
|---|---|---|---|---|---|
| Higher alcohols | 0.17 | 0.24 | 0.33 | 0.40 | 0.47 |
| $CH_3OH$ | -0.16 | -0.34 | -0.45 | -0.65 | -0.92 |
| $H_2$ | +0.01 | +0.02 | +0.03 | +0.03 | +0.04 |
| $CO_2$ | 0.29 | 0.47 | 0.53 | 0.51 | 0.56 |
| Selectivity (molar %) | 67 | 62 | 68 | 72 | 74 |

Alcohol compositions (wt %)

| | | | | | |
|---|---|---|---|---|---|
| $CH_3OH$ | 0 | 0 | 0 | 0 | 0 |
| $C_2H_5OH$ | 29 | 25 | 18 | 15 | 13 |
| $C_3H_7OH$ | 24 | 21 | 24 | 20 | 19 |
| $C_4H_9OH$ | 47 | 54 | 58 | 65 | 68 |

EXAMPLE 3

| Sample No. | 1 | 2 | 3 | 4 | 5 |
|---|---|---|---|---|---|
| Methanol L.H.S.V.$(h^{-1})$ | 0.35 | 0.70 | 1.06 | 1.41 | 1.76 |
| G.H.S.V.$(h^{-1})$ | 1700 | 2100 | 2000 | 1800 | 2000 |
| **Reactor inlet** | | | | | |
| $H_2$:CO | 0:100 | 0:100 | 0:100 | 0:100 | 0:100 |
| $H_2$ (Vol %) | 0 | 0 | 0 | 0 | 0 |
| CO (Vol %) | 91.4 | 86.7 | 80.3 | 73.1 | 70.2 |
| $CH_3OH$ (Vol %) | 8.6 | 13.5 | 19.8 | 26.8 | 29.6 |
| **Feed rate** (kg/h/kg cat.) | | | | | |
| $H_2$ | 0 | 0 | 0 | 0 | 0 |
| CO | 4.37 | 5.29 | 5.01 | 4.48 | 4.87 |
| $CH_3OH$ | 0.47 | 0.94 | 1.41 | 1.88 | 2.35 |
| **Product rate** (kg/h/kg cat.) | | | | | |
| $H_2$ | 0.01 | 0.04 | 0.05 | 0.06 | 0.07 |
| CO | 4.26 | 5.01 | 4.62 | 4.37 | 4.68 |
| $CH_3OH$ | 0.07 | 0.27 | 0.48 | 0.74 | 1.09 |
| $C_2H_5OH$ | 0.01 | 0.03 | 0.04 | 0.04 | 0.04 |
| $C_3H_2OH$ | 0.03 | 0.06 | 0.10 | 0.10 | 0.11 |
| $C_4H_9OH$ | 0.07 | 0.12 | 0.19 | 0.24 | 0.28 |
| $CH_4$ | 0.07 | 0.13 | 0.19 | 0.17 | 0.21 |
| $CO_2$ | 0.35 | 0.59 | 0.77 | 0.63 | 0.75 |
| **Conversions** (%) | | | | | |
| CO | 3 | 5 | 8 | 3 | 4 |
| $CH_3OH$ | 86 | 71 | 66 | 61 | 54 |

<u>EXAMPLE 3</u>   contd.

<u>Yields</u> (kg/h/kg cat.)

| Higher alcohols | 0.11 | 0.21 | 0.33 | 0.38 | 0.43 |
|---|---|---|---|---|---|
| $CH_3OH$ | -0.40 | -0.67 | -0.93 | -1.14 | -1.26 |
| $H_2$ | 0.01 | 0.04 | 0.05 | 0.06 | 0.07 |
| $CO_2$ | 0.35 | 0.59 | 0.77 | 0.63 | 0.75 |

<u>Selectivity</u> (molar %)   56   57   59   65   63

<u>Alcohol compositions</u> (wt %)

| $CH_3OH$ | 0 | 0 | 0 | 0 | 0 |
|---|---|---|---|---|---|
| $C_2H_5OH$ | 9 | 14 | 12 | 11 | 9 |
| $C_3H_7OH$ | 27 | 29 | 30 | 26 | 26 |
| $C_4H_9OH$ | 64 | 57 | 58 | 63 | 65 |

<u>EXAMPLE 3</u>   contd.

11

EXAMPLE 4

| Sample No. | | 1 | 2 | 3 | 4 |
|---|---|---|---|---|---|
| Methanol L.H.S.V.($h^{-1}$) | | 0.70 | 1.06 | 1.41 | 1.76 |
| G.H.S.V.($h^{-1}$) | | 2400 | 2400 | 2500 | 2700 |
| Reactor inlet | | | | | |
| $H_2$:CO | | 10:90 | 10:90 | 10:90 | 10:90 |
| $H_2$ | (Vol %) | 9.5 | 8.9 | 8.5 | 8.4 |
| CO | (Vol %) | 78.5 | 73.9 | 70.5 | 68.2 |
| $CH_3OH$ | (Vol %) | 12.1 | 17.1 | 20.9 | 23.6 |
| Feed rate (kg/h/kg cat.) | | | | | |
| $H_2$ | | 0.05 | 0.05 | 0.05 | 0.05 |
| CO | | 5.32 | 5.32 | 5.54 | 5.94 |
| $CH_3OH$ | | 0.94 | 1.41 | 1.88 | 2.35 |
| Product rate (kg/h/kg cat.) | | | | | |
| $H_2$ | | 0.14 | 0.15 | 0.16 | 0.19 |
| CO | | 4.56 | 4.62 | 4.76 | 5.26 |
| $CH_3OH$ | | 0.73 | 0.99 | 1.34 | 1.76 |
| $C_2H_5OH$ | | 0.05 | 0.05 | 0.06 | 0.06 |
| $C_3H_2OH$ | | 0.06 | 0.06 | 0.07 | 0.08 |
| $C_4H_9OH$ | | 0.14 | 0.50 | 0.47 | 0.30 |
| $CH_4$ | | 0.08 | 0.11 | 0.12 | 0.15 |
| $CO_2$ | | 0.36 | 0.43 | 0.47 | 0.56 |
| Conversions (%) | | | | | |
| CO | | 14 | 13 | 14 | 11 |
| $CH_3OH$ | | 22 | 30 | 29 | 25 |

EXAMPLE 4    contd.

Yields (kg/h/kg cat.)

| | | | | |
|---|---|---|---|---|
| Higher alcohols | 0.25 | 0.61 | 0.60 | 0.44 |
| $CH_3OH$ | -0.21 | -0.42 | -0.54 | -0.59 |
| $H_2$ | 0.09 | 0.10 | 0.11 | 0.14 |
| $CO_2$ | 0.36 | 0.43 | 0.47 | 0.56 |
| Selectivity (molar %) | 72 | 82 | 80 | 71 |

Alcohol compositions (wt %)

| | | | | |
|---|---|---|---|---|
| $CH_3OH$ | 0 | 0 | 0 | 0 |
| $C_2H_5OH$ | 20 | 8 | 10 | 14 |
| $C_3H_7OH$ | 24 | 10 | 12 | 18 |
| $C_4H_9OH$ | 56 | 82 | 78 | 68 |

* Pressure of 125 bar was used.

## Claims

1. A process for the production of higher alcohols characterised in that reacting a lower alcohol feed and a gas containing carbon monoxide and hydrogen with a composition in the range 20:80 to 0:100 by volume hydrogen to carbon monoxide over a heterogeneous solid phase alcohol synthesis catalyst under alcohol synthesis conditions, recycling unreacted alcohol feed and carbon monoxide and hydrogen after separating from the product higher alcohols, and maintaining a carbon monoxide partial pressure of at least 30 bar at the reactor exit.

2. A process according to claim 1, characterised in that the lower alcohol is methanol.

3. A process according to claim 1 or 2, characterised in that the CO partial pressure is in the range 40 to 300 bar.

4. A process according to any one of the preceding claims, characterised in that the lower alcohol feed rate is 0.2 litres/hour/litre of catalyst.

5. A process according to any one of the preceding claims, characterised in that the temperature in the reactor is from 240 to 350°C.

6. A process according to claim 5, characterised in that the temperature in the reactor is from 260 to 310°C.

7. A process according to any one of the preceding claims characterised in that the catalyst is molybdenum sulphide promoted with an alkali metal.

8. A process according to claim 7, characterised in that the catalyst is prepared as described in GB Patent Application 2 185 907 (European Patent Application No 0 235 886).

9. A mixed higher alcohol product whenever prepared by a process according to any one of the preceding claims.

FIG. 1

EP 0 311 297 A2

FIG. 2

EP 0 311 297 A2